# FASCICULE DE BREVET EUROPEEN

(11) **EP 1 113 758 B1**
(45) Date de publication et mention de la délivrance du brevet: **07.04.2004**
(21) Numéro de dépôt: 99941719.9
(22) Date de dépôt: 08.09.1999
(51) Int. Cl.: A61B 17/70

(54) **DISPOSITIF D'OSTEOSYNTHESE RACHIDIENNE POSTERIEURE**
VORRICHTUNG ZUR POSTERIOREN WIRBELSÄULENOSTEOSYNTHESE
POSTERIOR BACKBONE OSTEOSYNTHESIS DEVICE

(30) Priorité: 18.09.1998 FR 9811703
(43) Date de publication de la demande: 11.07.2001
(73) Titulaire: EUROSURGICAL, 62217 Beaurains (FR)
(72) Inventeur: STEIB, Jean-Paul, F-67100 Strasbourg (FR); GALLARD, Emeric, F-79160 Fenioux (FR); VIART, Guy, F-62128 Saint Léger (FR); FREUND, Jean-Pierre, F-67000 Strasbourg (FR)
(74) Mandataire: Schmitt, John
(86) Numéro de dépôt international: PCT/FR1999/002136
(87) Numéro de publication internationale: WO 2000/016710

(56) Documents cités:
- EP-A- 0 558 121
- FR-A- 2 642 642
- FR-A- 2 697 992
- US-A- 5 413 576

## Description

La présente invention a pour objet un dispositf d'ostéosynthèse rachidienne postérieure, destiné à assurer une liaison transversale entre deux tiges vertébrales s'étendant le long d'un segment rachidien.

Un tel dispositif est du type comprenant une paire de crochets d'appui - latéral sur une vertèbre, fixés à un moyen de liaison entre eux.

La demande de brevet internationale WO 97/25931 décrit un dispositif de liaison transversale postérieure dans lequel deux crochets sont reliés par un élément tubulaire traversant une pièce centrale disposée au-dessus de la crête épineuse de la vertèbre, cette pièce centrale servant d'appui à des tiges longitudinales.

Lorsque les chirurgiens orthopédistes veulent corriger des scolioses, ils peuvent utiliser des vis pédiculaires au niveau des vertèbres lombaires, grâce au fait que les dimensions des pédicules de ces vertèbres sont suffisantes pour des vis. En revanche, au niveau des vertèbres thoraciques, qui sont plus minces que les vertèbres lombaires, la place disponible est rarement suffisante pour des vis, ce qui conduit les chirurgiens à utiliser des crochets pédiculaires associés à des crochets transverses.

Or, lorsque la scoliose est importante, le chirurgien éprouve des difficultés pour déplacer la vertèbre jusqu'à la position voulue à l'aide de crochets. En effet, la vertèbre doit être déplacée en translation dans le plan frontal ainsi qu'en rotation dans le plan sagittal en pivotant par conséquent sur elle-même. Il en résulte un risque de glissement des crochets sur lesquels s'appuie le chirurgien. En résumé, si le chirurgien doit exercer uniquement un effort de rappel sur la vertèbre, un seul crochet peut suffire. Si par contre, la vertèbre doit en outre tourner sur elle-même, le chirurgien pose un second crochet pédiculo-transverse de l'autre côté du pédicule, une tige de liaison traversant les deux crochets, ce qui diminue le risque de glissement de ceux-ci. Il n'en reste pas moins que ce risque existe, en raison de la prise insuffisante assurée par les crochets.

Par ailleurs, les crochets sont ajustés au mieux sur les vertèbres sans y être fixés. Il en résulte un jeu appréciable et donc un médiocre ancrage osseux, qui n'autorisent pas d'efforts trop élevés du couple de redressement. De plus, la pose des crochets et des vis est longue et augmente indésirablement la durée de l'intervention chirurgicale.

En définitive, les différents crochets pédiculaires, transversaires et laminaires, ne sont pas efficaces dans les manoeuvres de correction en rotation de la position des vertèbres, leur action sur celles-ci étant limitée à des déplacements dans les sens postéro-antérieur ou antéro-postérieur. En outre, la lame des crochets laminaires est dans le canal médullaire en contact avec la dure-mère.

Les vis vertébrales des instrumentations connues présentent aussi un risque non négligeable de toucher la moëlle épinière lors de leur pose par le chirurgien, outre le fait qu'elles exigent des pédicules de taille suffisante.

L'invention a pour but de proposer un dispositif d'ostéosynthèse rachidenne postérieure du type mentionné ci-dessus, qui soit réalisé de manière à permettre une prise solide des crochets sur la vertèbre, suffisante pour écarter tout risque de glissement ou de basculement des crochets lors d'un mouvement de rotation imprimé à la vertèbre pour corriger une scoliose.

Conformément à l'invention, le corps des crochets est conformé pour prendre appui sur des processus transverses de la vertèbre préalablement réséqués, et le moyen de liaison est constitué par une paire de tiges parallèles solidarisées ensemble, cintrées élastiquement en formant un arc transversal, dont les extrémités s'engagent dans des alésages agencés dans les crochets, et des moyens sont prévus pour fixer les crochets aux extrémités des tiges de l'arc tranversal dans des positions générant des couples de rappel élastique par l'arc transversal des crochets en appui sur les processus transverses.

Pour préparer la pose d'un tel dispositif, le chirurgien procède à la coupe des deux processus (ou apophyses) transverses à l'endroit adéquat, ce qui dégage un espace accessible entre la face de coupe de chaque processus transverse et la côte correspondante lorsqu'il s'agit d'une vertèbre thoracique. En effet, si la première application du dispositif selon l'invention vise les vertèbres thoraciques, ce dispositif peut également tout aussi bien être posé sur les autres vertèbres, en particulier les vertèbres lombaires.

Une fois les deux crochets mis en place sur l'arc transversal, cintré élastiquement, le chirurgien serre ensuite, à l'aide d'une pince appropriée, les deux crochets en appui sur les processus transverses, pour les faire glisser sur l'arc transversal en les rapprochant l'un de l'autre, ce qui accentue la courbure des tiges de l'arc qui reste dans son domaine élastique. Les crochets sont ensuite verrouillés sur l'arc. Celui-ci exerce alors sur les crochets un couple de rappel qui les maintient fermement appliqués sur les processus transverses. On réalise ainsi un assemblage solide dont les crochets ne dérapent pas sur la vertèbre lors des efforts qui leur sont imprimés par le chirurgien pour faire pivoter la vertèbre, et qui restent ensuite en place sans jeu.

L'arc vertébral est avantageusement constitué de deux tiges cylindriques soudées, au lieu d'une seule. En effet, la mise en oeuvre d'une seule tige entrainerait un risque de rotation d'un crochet sur la tige par rapport à l'autre, deux tiges parallèles et solidaires empêchant par contre tout basculement des crochets sur l'arc transversal.

Les corps des crochets sont quelconques mais délimitent de préférence un canal en U adapté pour recevoir la tige vertébrale et sont munis de moyens de verrouillage appropriés de la tige sur le crochet.

Le dispositif selon l'invention permet d'arthrodéser deux ou plusieurs vertèbres avec au moins deux dispositifs de liaison de ce type associés à deux tiges vertébrales.

Suivant un mode de réalisation préféré, les alésages des crochets ont une section oblongue complémentaire de celle des tiges cylindriques de l'arc transversal.

Suivant un mode de réalisation de l'invention, chaque crochet comprend un corps conformé pour recevoir la tige vertébrale correspondante, et une hampe latérale adaptée pour pouvoir prendre appui sur le processus transverse, et l'alésage oblong est ménagé à la jonction entre le corps et la hampe pour être traversé par l'extrémité de l'arc transversal ; un trou taraudé agencé dans la hampe débouche dans le trou oblong, ce trou taraudé étant adapté pour être équipé d'une vis de blocage du crochet sur l'arc transversal.

Les deux tiges de l'arc transversal peuvent être solidarisées par tout moyen approprié, par exemple par soudage laser.

D'autres particularités et avantages de l'invention apparaîtront au cours de la description qui va suivre, faite en référence aux dessins annexés qui en illustrent plusieurs formes de réalisation à titres d'exemples non limitatifs.
- La Figure 1 est une vue en élévation schématique d'une instrumentation d'ostéosynthèse rachidienne pourvue de dispositifs de liaison transversale selon l'invention, mis en place sur un segment rachidien affecté d'une scoliose avant correction de celle-ci.
- la Figure 2 est une vue analogue à la Figure 1 montrant l'instrumentation complétée sur le segment rachidien dont la scoliose a été corrigée.
- La Figure 3 est une vue en perspective sensiblement à l'échelle, d'une première forme de réalisation du dispositif d'ostéosynthèse rachidienne postérieure conforme à l'invention implanté sur une vertèbre.
- La Figure 4 est une vue en coupe longitudinale à échelle agrandie du dispositif d'ostéosynthèse de la Figure 3.
- La Figure 5 est une vue en coupe transversale suivant 5-5 de la Figure 4.
- Les Figures 6, 7 et 8 sont des vues en coupe longitudinale analogues à la Figure 5 illustrant une séquence d'implantation du dispositif d'ostéosynthèse sur une vertèbre thoracique.
- La Figure 9 est une vue en coupe transversale suivant 9-9 de la Figure 4.
- La Figure 10 est une vue en coupe partielle à échelle agrandie d'un crochet selon une seconde forme de réalisation du dispositif.
- La Figure 11 est une vue en perspective partielle du dispositif d'ostéosynthèse selon la seconde forme de réalisation de la Figure 10, mis en place sur une vertèbre.
- La Figure 12 est une vue en perspective à échelle agrandie d'une troisième forme de réalisation du crochet du dispositif.
- La Figure 13 est une vue en coupe transversale à échelle agrandie, dans un plan horizontal, d'une quatrième forme de réalisation du dispositif d'ostéosynthèse implanté sur une vertèbre.
- La Figure 14 est une vue analogue à la Figure 12 illustrant une cinquième forme de réalisation du dispositif.
- La Figure 15 est une vue en coupe partielle à échelle agrandie suivant la ligne 15-15 de la Figure 14.
- La Figure 16 est une vue mi-coupe mi-élévation partielle d'une sixième forme de réalisation du dispositif.
- La Figure 17 est une vue en coupe suivant 17-17 de la Figure 16.
- La Figure 18 est une vue en coupe transversale suivant 18-18 de la Figure 16.
- La Figure 19 est une vue en perspective de l'arc transversal du dispositif des Figures 16 à 18.
- La Figure 20 est une vue en perspective d'un crochet selon une septième forme de réalisation.
- La Figure 21 est une vue en coupe du crochet selon 21-21 de la Figure 20.

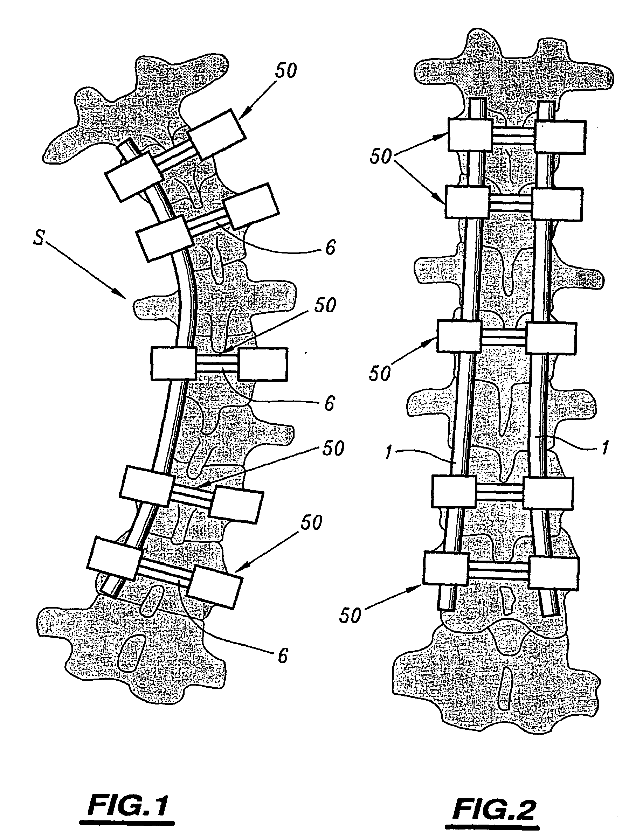

L'instrumentation d'ostéosynthèse rachidienne postérieure illustrée aux Figures 1 et 2 est destinée à corriger des déviations vertébrales telles qu'une scoliose d'un segment rachidien S, pour redresser celui-ci dans la position de la Figure 2.

Cette instrumentation comprend deux tiges vertébrales longitudinales postérieures 1, s'étendant le long du segment S de chaque côté des épineuses vertébrales, ainsi qu'un ensemble de dispositifs 50 de liaison transversale entre les tiges 1, convenablement répartis entre les extrémités de celles-ci afin de conférer une rigidité suffisante au montage.

Chaque dispositif 50 comprend une paire de crochets transverses 2 traversés chacun par une tige vertébrale 1, et un arc transversal 6 de liaison entre les crochets 2.

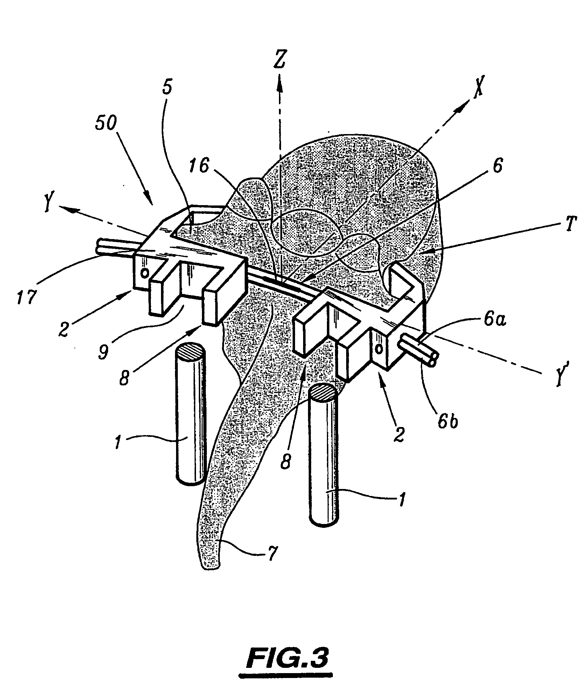

Le dispositif d'ostéosynthèse rachidienne postérieure illustré aux Figures 3 à 9 est destiné à réaliser une liaison transversale entre deux tiges vertébrales 1 s'étendant le long d'un segment rachidien d'au moins deux étages vertébraux.

Chaque dispositif 50 comprend une paire de crochets 2 d'appui latéral sur une vertèbre, qui peut être une vertèbre thoracique T disposée entre deux côtes 3 de la cage thoracique comme représenté aux Figures 2 à 8, ou bien une vertèbre d'un segment rachidien quelconque telle qu'une vertèbre lombaire (Figures 1 et 2). Ces crochets 2 sont disposés latéralement à la vertèbre, en étant agencés de manière à pouvoir prendre appui sur des faces planes 4 préalablement reséquées des processus (ou apophyses) transverses 5 de la vertèbre T. Le dispositif est complété par un arc transversal 6 de liaison entre les deux crochets 2, dont les extrémités opposées sont solidarisées avec ces derniers. L'arc transversal 6 prend appui dans sa zone centrale sur la base de l'épineuse 7 de la vertèbre T.

Chaque crochet 2 comprend un corps 8 à section sensiblement en U et délimitant un canal intérieur 9 dans lequel peut être disposée une tige vertébrale 1. Le corps 8 en U peut être réalisé selon de nombreuses variantes, par exemple comme décrit dans le brevet français 2 697 992 (92 13 868) au nom d'EUROSURGICAL. Chaque crochet 2 est muni de moyens de verrouillage de la tige vertébrale 1 dans le corps 8, ces moyens pouvant être par exemple similaires à ceux décrits dans le brevet français précité.

Chaque corps 8 est prolongé par une hampe latérale 11, prévue pour être positionnée en regard de la face réséquée 4 du processus transverse 5 afin de pouvoir prendre appui sur celle-ci. A cet effet la hampe 11 s'étend dans une direction à peu près parallèle à un plan contenant l'axe longitudinal de la tige vertébrale 1, et se termine par une extrémité recourbée 12 formant lame d'accrochage. La lame 12 est conformée pour pouvoir s'étendre sous le processus transverse 5, tandis que le corps 8 est disposé postérieurement à ce processus transverse, plus près de l'épineuse 7. La lame terminale 12 formant crochet est rectiligne et forme avec la hampe 11 un angle obtus, pouvant varier sensiblement selon la conformation anatomique de la vertèbre.

Dans chaque crochet 2 est ménagé un alésage 13, de section oblongue, qui s'étend à la jonction de la hampe 11 et du corps 8 dans la direction transversale, sensiblement perpendiculaire à l'axe longitudinal du canal 9. Dans cet alésage oblong 13 débouche un trou taraudé 14 usiné dans une partie latérale 15 saillant du corps 8 et constituant la base de la hampe 11.

L'arc transversal 6 est constitué de deux tiges métalliques 6a, 6b parallèles, solidarisées ensemble par exemple par soudage au laser dans leur zone centrale 16. Les deux tiges 6a, 6b sont réalisées en un matériau métallique approprié, cintrées élastiquement et s'étendent sous les corps 8 des crochets 2 en formant un arc dont les extrémités opposées 6c s'engagent dans les alésages oblongs 13 dont la section transversale est complémentaire du pourtour des deux tiges 6a, 6b.

Les crochets 2 sont verrouillés sur les extrémités 6c de l'arc 6 par des vis pointeau 17 venant se visser dans les trous 14 jusqu'à ce que leur pointe 17a s'enfonce entre les deux tiges 6a, 6b et verrouille ainsi les crochets respectifs 2 sur l'arc transversal 6, après que le chirurgien ait positionné convenablement les crochets 2 sur l'arc 6.

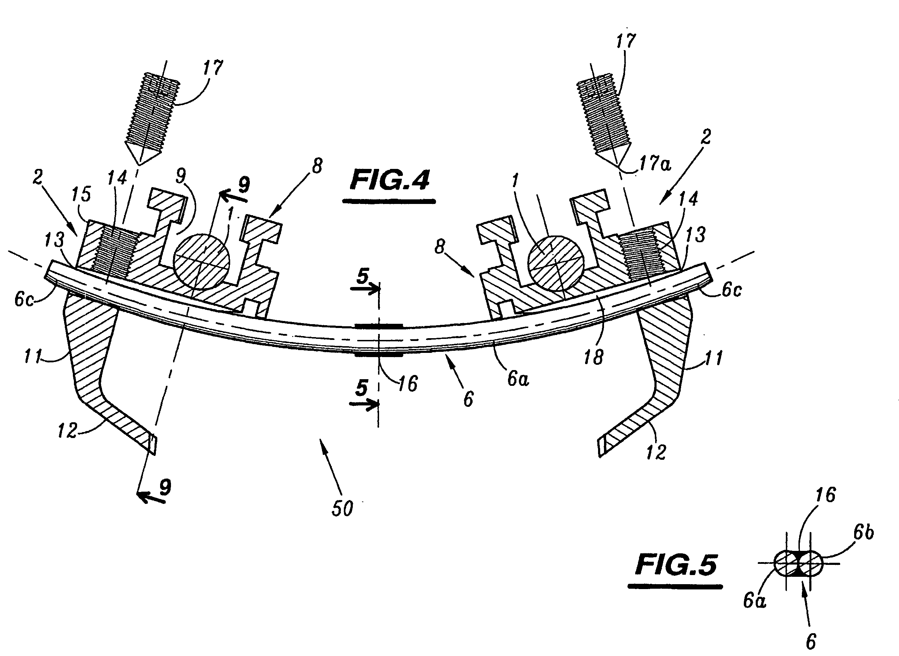

Dans chaque crochet 2, l'alésage oblong 13 est prolongé par un dégagement longitudinal 18 (Figure 4) usiné dans la face du corps 8 opposé à son canal 9, et constituant une sorte de glissière facilitant la mise en place de l'arc transversal 6.

Les processus transverses 5 subissent une ablation partielle préalable telle que la hampe 11 et la lame de crochet 12 puissent respectivement prendre appui contre la portion transverse restante 5 et atteindre les pédicules de la vertèbre T par voie latérale.

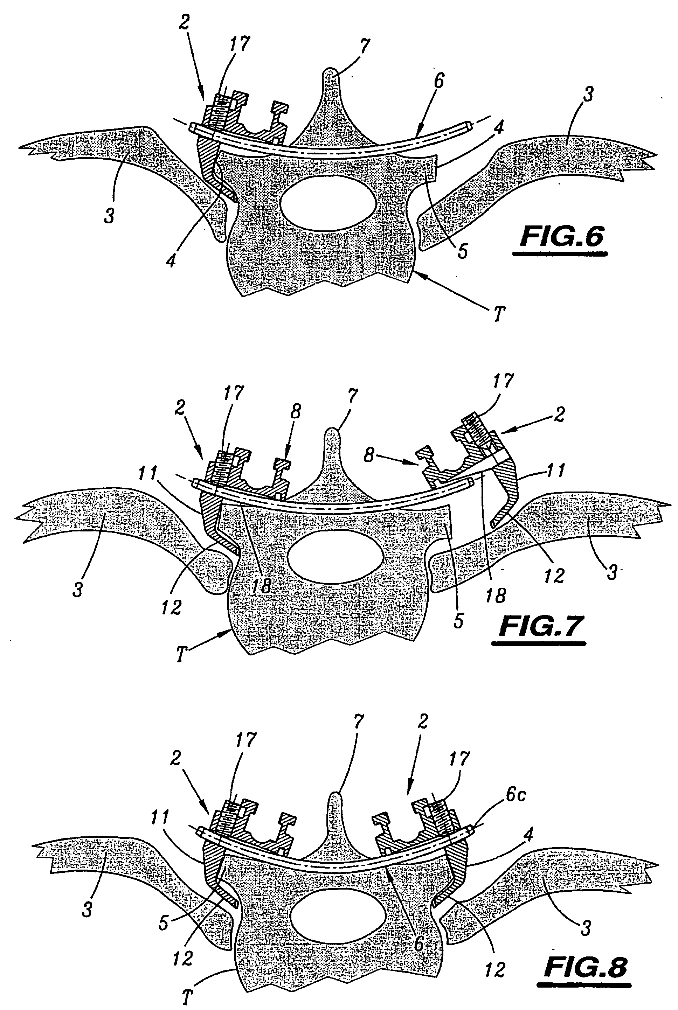

Les figures 6 à 8 illustrent une séquence opératoire d'implantation du dispositif de liaison transversale selon l'invention sur une vertèbre thoracique T.

Après avoir procédé à l'ablation partielle des processus transverses 5 afin d'obtenir des faces réséquées 4, le chirurgien enfile un crochet 2 d'un côté de l'arc transversal 6 sur lequel il le positionne en serrant la vis 17. Le chirurgien introduit ensuite l'ensemble crochet 2 et arc 6 sur la vertèbre T en contournant la portion de transverse restante 5 du côté considéré, comme illustré à la Figure 6. Suivant les cas cliniques, la hampe 11 et/ou la lame d'extrémité 12, du crochet 2 viennent en contact avec la portion de transverse restante 5 et/ou avec les pédicules de la vertèbre T. Le chirurgien enfile le second crochet 2 à l'autre extrémité 6c de l'arc transversal 6 (Figure 7), en introduisant l'extrémité 6c dans l'alésage ablong 13 jusqu'à ce que le crochet 2 vienne à son tour en butée latérale avec l'autre pédicule de la vertèbre T et/ou l'autre portion de transverse restante 5.

La dernière manipulation du chirurgien, illustrée à la Figure 8, consiste à faire glisser le second crochet 2 sur l'arc transversal 6 en rapprochant les deux crochets l'un de l'autre. Les lames terminales 12 étant en contact bilatéral à hauteur des pédicules de la vertèbre T, le glissement des crochets sur l'arc 6 est obtenu par le chirurgien au moyen d'une pince non représentée qui permet de rapprocher les deux crochets, oblige l'arc transversal 6 à se cintrer davantage tout en restant dans son domaine élastique (Figure 8). Après vissage des vis de blocage de chaque crochet sur l'arc 6, ce dernier exerce alors sur les crochets 2 des couples de rappel correspondant à sa tendance à retrouver sa courbure initiale. Ces couples de rappel tendent à resserrer les crochets 2 contre les pédicules et/ou les portions de transverses restantes 5, et donc à renforcer le maintien en place des crochets 2.

Le dispositif de liaison transversale assure alors des ancrages osseux solidaires de manière entièrement satisfaisante de la vertèbre T sur laquelle il est implanté, et aisés à mettre en oeuvre.

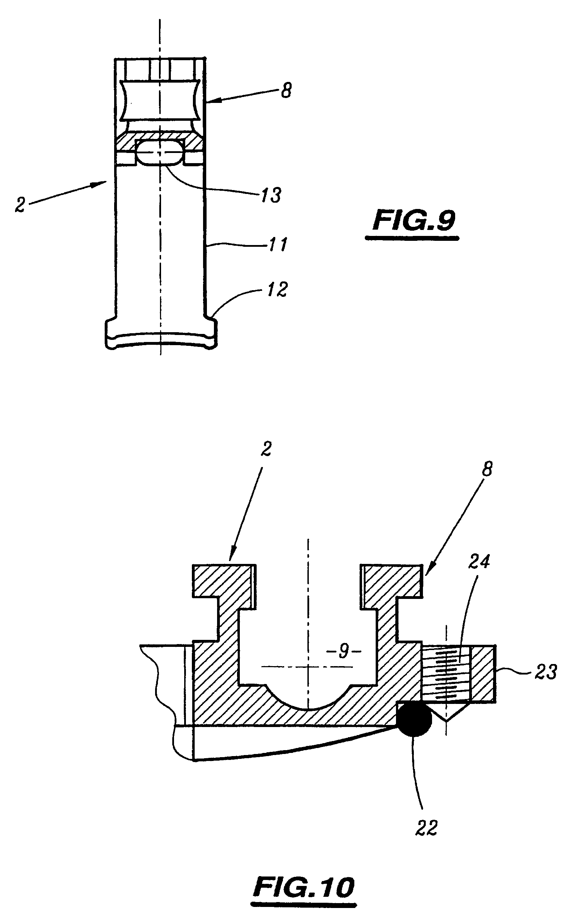

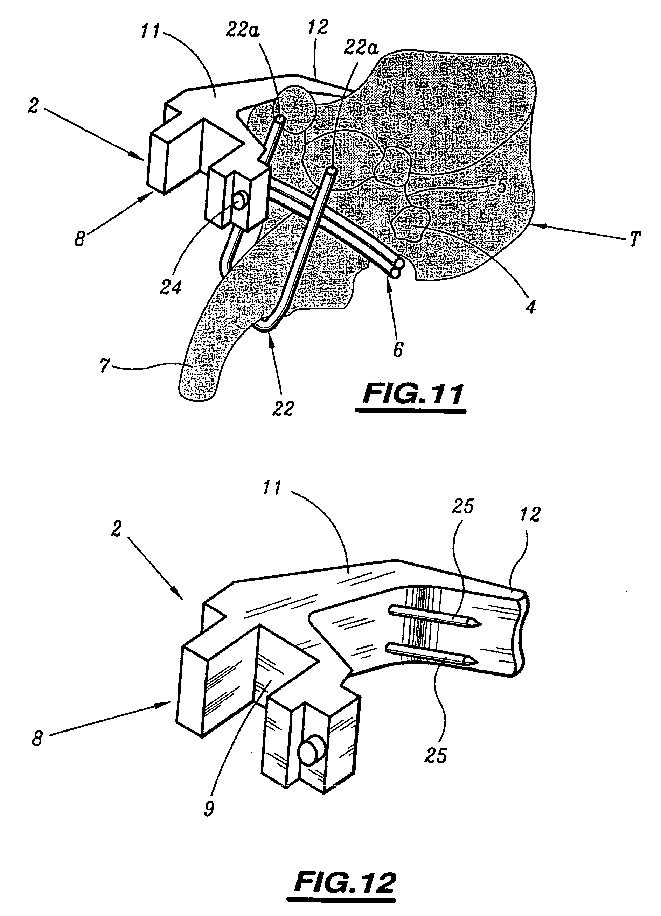

Dans la seconde forme de réalisation du dispositif illustrée aux Figures 10 et 11, le dispositif est muni d'un arc secondaire 22 constitué d'un fil profilé sensiblement en U, adapté pour passer sous la crête épineuse 7 de la vertèbre T. Les branches 22a de l'arc secondaire 22 viennent prendre appui sous les corps 8 des crochets 2 en exerçant sur l'arc transversal 6 des couples de rappel permettant de le plaquer sur la crête épineuse 7. Plus précisément, chaque branche 6a prend appui sur une excroissance latérale 23 du corps 8 du crochet 2 ainsi que sur la paroi du corps 8, en y étant maintenue par des moyens de blocage appropriés tels qu'une vis pointeau 24 insérée dans chaque excroissance 23.

Cette variante de réalisation permet d'améliorer la tenue du dispositif, notamment au niveau de la rotation autour de l'axe Y-Y' (Figure 3), cet axe étant horizontal et situé dans le plan frontal. L'arc secondaire 22, par les couples de rappel exercés sur l'arc transversal 6, plaque celui-ci contre la partie haute de la crête épineuse 7 ainsi que l'ensemble du dispositif sur la vertèbre et s'oppose par conséquent à un basculement autour de l'axe Y-Y' par rapport à la vertèbre.

Le dispositif présente également l'avantage d'être en liaison amovible et permanente avec la vertèbre. En outre, l'utilisation d'un arc élastique 6 avec une assez forte déformation potentielle, permet aux crochets 2 de rester en contact avec l'os malgré une éventuelle variation de l'effort à l'interface os-crochet 2, d'où pourrait résulter une relaxation de l'os sous l'effort de serrage par exemple.

Le montage peut s'accompagner de greffes osseuses, sans obligation d'ôter ultérieurement le dispositif. Toutefois, un tel démontage est aisé à réaliser par dévissage des vis de serrage 17.

Dans le troisième mode de réalisation du dispositif, illustré à la Figure 12, chaque crochet est équipé de pointes 25, au nombre par exemple de deux comme représenté, qui font saillie de la hampe 11 entre le corps 8 et la lame recourbée 12. Ces éléments pointus 25 viennent pénétrer dans l'os spongieux de la portion des transverses restantes 5 correspondantes, lorsque les crochets 2 sont mis en place sur la vertèbre T de la façon décrite précédemment. Cette forme de réalisation constitue une autre manière d'améliorer la tenue du dispositif sur la vertèbre T, en particulier pour empêcher toute rotation autour de l'axe Y-Y' du dispositif par rapport à la vertèbre.

Dans les divers modes de réalisation décrits précédemment, le corps de la hampe 11 s'appuie sur la face tronquée 4 du processus transverse 5 lorsque le dispositif est mis en place, tandis que l'extrémité courbée 12 formant lame est adaptée pour s'étendre le long dudit processus transverse 5.

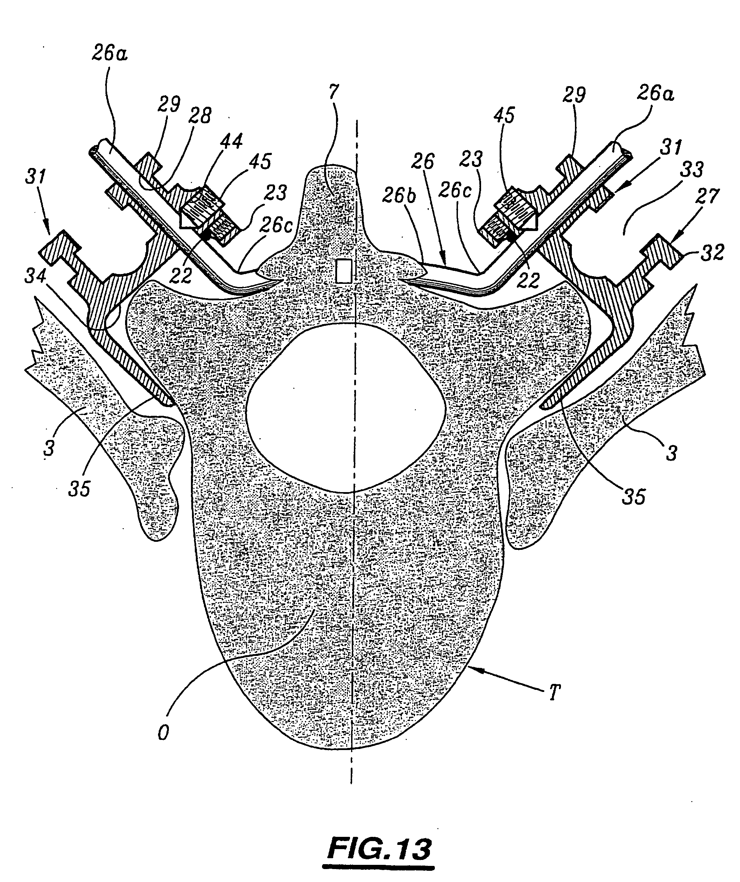

La quatrième forme de réalisation du dispositif illustrée à la Figure 13, diffère des précédentes par le fait que l'arc transversal 26, formé de deux fils comme précédemment, présente une triple courbure dans un plan horizontal afin de lui permettre de mieux s'adapter aux formes anatomiques de l'arc postérieur de la vertèbre T. Plus précisément, entre ses extrémités rectilignes 26a insérées dans les crochets respectifs 27, l'arc transversal 26 comporte une partie centrale concave 26b dont la concavité épouse sensiblement la convexité de l'arc postérieur de la vertèbre, et deux parties latérales convexes 26c de raccordement de la partie centrale 26b et des parties terminales rectilignes 26a.

Chaque extrémité 26a de l'arc 26 est logée dans un alésage 28 ménagé dans une branche 29 du corps 31 du crochet 27, la seconde branche 32 délimitant avec la branche 29 le canal en U 33 de logement de la tige vertébrale 1. Le fond 34 du corps 31 s'étend en regard du processus transverse reséqué 5 lorsque le dispositif est mis en place. Le corps 31 se prolonge latéralement par une hampe 35 sensiblement rectiligne qui s'étend à peu près parallèlement à la branche 29. Lorsque le dispositif est monté sur la vertèbre T, le corps 31 s'appuie donc sur la face réséquée 4 du processus transverse 5 et la hampe latérale 35 s'étend entre la côte adjacente 3 et le côté du processus transverse 5.

Un trou taraudé 44 est agencé dans la branche 29 du corps 31 et débouche dans l'alésage oblong 28. Le trou 44 est adapté pour recevoir une vis 45 de blocage du crochet correspondant 27 sur l'arc transversal 26 dans la position voulue après orientation et glissement du corps 31 sur l'extrémité rectiligne 26a de l'arc transversal 26.

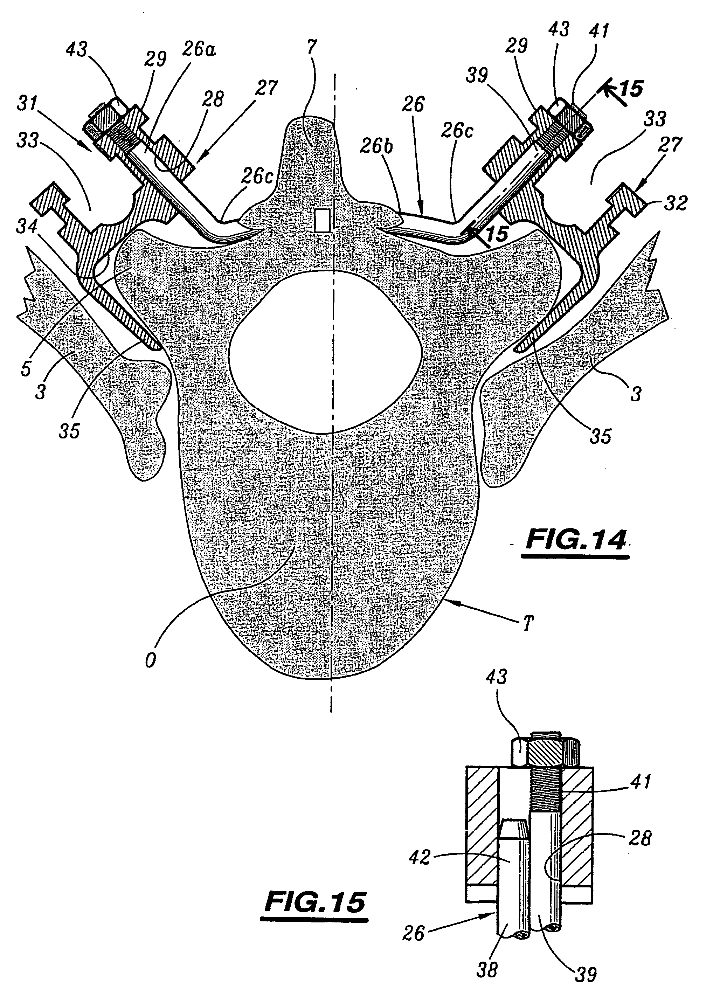

Dans la cinquième forme de réalisation du dispositif (Fig. 14 et 15) l'arc transversal 26 est constitué de deux fils métalliques 38, 39 dont l'un, par exemple le fil 39, comporte une extrémité filetée 41 s'étendant au-delà de l'extrémité lisse 42 du fil 38 de manière à faire saillie extérieurement à l'alésage 28. Cette extrémité filetée 41 est munie d'un écrou 43 de blocage du crochet correspondant 27 sur l'arc transversal 26.

Bien entendu, l'alésage 28 a une section oblongue adaptée à celle des deux fils accolés 38, 39. Cet alésage oblong 28 s'étend dans une direction à peu près parallèle à celle de la hampe latérale 35. Les écrous 43 permettent à la fois un glissement et un blocage des deux crochets 27 sur l'arc transversal 26.

Les deux réalisations des Figures 13 à 15 permettent de limiter l'encombrement postérieur du dispositif grâce à l'emplacement des corps 31 des crochets 27, qui sont décalés latéralement en regard des faces 4 des processus transverses 5, et ce grâce à la triple courbure de l'arc transversal 26. En outre, les corps 31 des crochets sont par là même éloignés du centre instantané de rotation ○ de la vertèbre T, ce qui augmente le bras de levier et donc, réduit les efforts induits à développer lors d'un couple de redressement de la vertèbre.

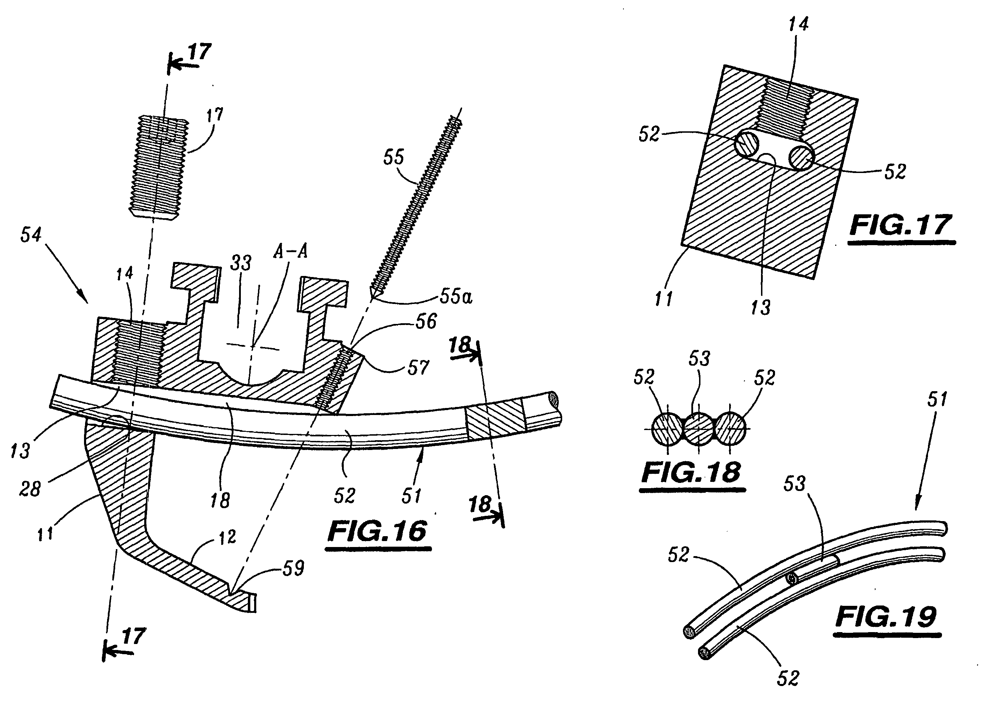

Selon la sixième forme de réalisation représentée aux Figures 16 à 19, l'arc transversal 51 est constitué de trois tiges dont deux tiges longues extérieures 52 et une tige centrale courte 53 soudée aux deux autres, par exemple au laser. Chaque crochet 54 associé est équipé d'une vis 55 pouvant être vissée dans un alésage taraudé 56 usiné dans un renfort latéral 57 du corps du crochet 54. L'axe longitudinal de l'alésage 56 est incliné sur l'axe longitudinal A-A du canal 33 du crochet 54 et la vis 55 a une longueur telle qu'après avoir été vissée dans l'alésage 56, elle peut traverser de part en part le processus transverse correspondant 5 et que sa pointe 55a vient en butée dans une empreinte 59 formée dans l'extrémité de la lame recourbée 12.

L'arc transversal 51 permet, grâce à l'écartement entre les deux tiges 52, le passage entre celles-ci de la vis 55, qui traverse la portion de processus transverse restante 5 dans une direction sensiblement perpendiculaire à l'axe principal dudit processus transverse.

En outre, ce mode de réalisation permet, grâce à la vis supplémentaire 55, de mieux rigidifier le dispositif de liaison transversale par rapport à la vertèbre T, notamment en ce qui concerne la rotation autour de l'axe Y-Y' et la translation suivant l'axe Z. La vis de serrage 17 assure toujours le blocage du crochet 54 par rapport à l'arc 51, même si les tiges 52 ne sont plus jointives comme dans le dispositif des Figures 3 à 9.

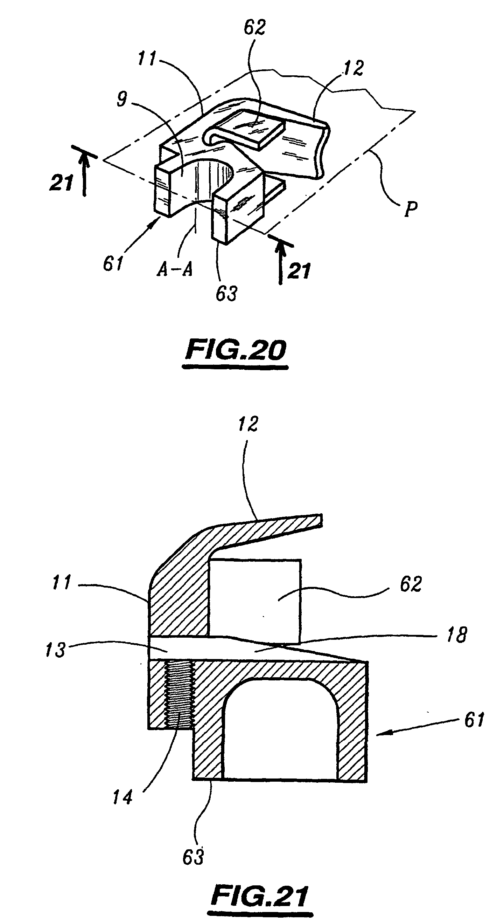

La forme de réalisation du crochet 61 illustrée aux Figures 20 et 21, diffère des précédentes par le fait que le crochet 61 est muni de deux flans 62 formant des pattes parallèles, disposées symétriquement par rapport à un plan P de symétrie du crochet 61 et qui s'étendent à partir de sa hampe 11 entre son corps 63 et la lame recourbée 12. Les flans 62 s'étendent donc également à peu près perpendiculairement à l'axe longitudinal A-A du canal 9 en U du crochet 61. Certains au moins des crochets des dispositifs de liaison transversale faisant partie de l'instrumentation d'ostéosynthèse visée par l'invention peuvent être ainsi modifiés.

L'adjonction des flans 62 évite tout risque de translation du dispositif de liaison transversale de bas en haut ou de haut en bas, c'est-à-dire suivant l'axe Z. Les portions de tranverses restantes 5 sont alors "noyées" dans une petite cavité délimitée par les flans 62, la lame 12 et la base du corps 63 du crochet 61.

L'invention n'est pas limitée aux modes de réalisation décrits et peut comporter de nombreuses variantes d'exécution. Par exemple, les éléments pointus 25 peuvent être en nombre variable, au minimum un.

Il convient en outre de noter que les modes de réalisation des Figures 10 à 12 peuvent être combinés avec les variantes des Figures 13 à 15.

## Revendications

1. Dispositif d'ostéosynthèse rachidienne postérieure, comprenant au moins deux paires de crochets (2, 27, 54, 61) d'appui latéral sur des vertèbres (T) fixés à des moyens de liaison transversale entre eux, et deux tiges vertébrales (1) adaptées pour être reçues dans des corps (8, 31) des crochets et pour s'étendre le long d'un segment rachidien (S), **caractérisé en ce que** le corps (8, 31) des crochets (2, 27, 54, 61) est conformé d'une hampe (11, 35) adaptée pour prendre appui sur des processus transverses (5) de la vertèbre (T) préalablement réséqués et **en ce que** lesdits moyens de liaison sont constitués pour chaque paire de crochets, par une paire de tiges parallèles (6a, 6b) solidarisées ensemble, cintrées élastiquement en formant un arc transversal (6, 26, 51) dont les extrémités (6c) s'engagent dans des alésages (13) agencés dans les crochets (2), et **en ce que** des moyens sont prévus pour fixer les crochets aux extrémités (6c) des tiges de l'arc transversal, dans des positions générant des couples de rappel élastique par l'arc transversal (6, 26, 51) des crochets en appui sur les processus transverses.

2. Dispositif selon la revendication 1, **caractérisé en ce que** les alésages (13) ont une section oblongue complémentaire de celle des tiges (6a, 6b) de l'arc transversal (6).

3. Dispositif selon la revendication 2, **caractérisé en ce que** le corps (8) de chaque crochet (2) est conformé pour recevoir la tige vertébrale correspondante (1), et une hampe latérale (11) adaptée pour pouvoir prendre appui sur le processus transverse (5), l'alésage oblong (13) est ménagé à la jonction entre le corps et la hampe pour être transversé par l'extrémité (6c) de l'arc transversal (6), et un trou taraudé (14) agencé dans la hampe (11) débouche dans le trou oblong (13), ce trou taraudé étant adapté pour être équipé d'une vis (17) de blocage du crochet sur l'arc transversal.

4. Dispositif selon l'une des revendications 1 à 3, **caractérisé en ce que** les deux tiges (6a) de l'arc transversal (6) sont solidarisées par soudage (16).

5. Dispositif selon la revendication 3, **caractérisé en ce que** la hampe (11) est pourvue d'une extrémité courbée (12) formant lame, adaptée pour s'étendre le long du processus transverse réséqué (5), tandis que le reste de la hampe est agencé pour s'appuyer sur la face tronquée (4) du processus transverse (5).

6. Dispositif selon l'une des revendications 1 à 5, **caractérisé en ce qu'**il est muni d'un arc secondaire (22) constitué d'un fil sensiblement en U adapté pour passer sous la crête épineuse (7) de la vertèbre (T), et dont les branches (22a) viennent prendre appui sous les corps (8) des crochets (2) en exerçant sur l'arc transversal des couples permettant de le plaquer sur la crête épineuse.

7. Dispositif selon la revendication 6, **caractérisé en ce que** les branches (22a) de l'arc secondaire (22) s'appuient sur des excroissances latérales (23) des corps (8) des crochets (2), et y sont maintenus par des moyens de blocage, tels que des vis (24) insérées dans lesdites excroissances.

8. Dispositif selon la revendication 3, **caractérisé en ce que** chaque crochet (2) est pourvu d'au moins une pointe (25) faisant saillie de sa hampe (11) afin de pouvoir s'enfoncer dans le processus transverse (5).

9. Dispositif selon l'une des revendications 1 à 8, **caractérisé en ce que** l'arc transversal (26) présente une triple courbure dans un plan horizontal pour s'adapter aux formes anatomiques de l'arc postérieur de la vertèbre, à savoir, entre des extrémités rectilignes (26a) insérées dans les crochets (27) respectifs, une partie centrale concave (26b) et deux parties latérales convexes (26c) de raccordement aux extrémités.

10. Dispositif selon la revendication 9, **caractérisée en ce que** chaque extrémité (26a) de l'arc transversal (26) est logée dans un alésage (28) ménagé dans une branche (29) du corps (31) du crochet (27), ce corps étant prolongé latéralement par une hampe (35) de sorte qu'une fois monté sur la vertèbre (T), le corps s'appuie sur la face réséquée (4) du processus transverse (5) et la hampe s'étend le long de celui-ci.

11. Dispositif selon la revendication 10, **caractérisé en ce que** dans ladite branche (29) du corps (31) est agencé un trou taraudé (44) débouchant dans l'alésage (28) et adapté pour recevoir une vis (45) de blocage du crochet (27) sur l'arc transversal (26).

12. Dispositif selon la revendication 10, **caractérisé en ce que** l'arc transversal (26) est formé de deux fils métalliques (38, 39) dont l'un au moins (39) comporte une extrémité filetée (41) s'étendant au-delà de l'extrémité du fil contigu (38) de manière à faire saillie extérieurement à l'alésage (28) du corps du crochet, et cette extrémité filetée est munie d'un écrou (43) de blocage du crochet sur l'arc transversal (26).

13. Dispositif selon la revendication 5, **caractérisé en ce que** l'arc transversal (51) est constitué de trois tiges dont deux tiges longues extérieures (52) et une tige centrale courte (53) soudée aux deux autres, est **en ce que** le crochet (54) est équipé d'une vis (55) positionnée pour traverser un renfort latéral (57) du corps du crochet et le processus transverse correspondant (5), avec sa pointe (55a) venant en butée dans une empreinte (59) de l'extrémité courbée (12) formant lame du crochet

14. Dispositif selon la revendication 5, **caractérisé en ce que** certains crochets au moins (61) sont munis de deux flans (62) disposés symétriquement par rapport à un plan de symétrie (P) du crochet, et qui s'étendent à partir de sa hampe (11) entre son corps (63) et la lame recourbée (12).

## Patentansprüche

1. Vorrichtung zur operativen wirbelsäulenbezüglichen rückwärtigen Knochenvereinigung bei nicht möglicher Reposition, umfassend wenigstens zwei Paare von Haken (2, 27, 54, 61) zum seitlichen Aufliegen auf Wirbeln (T), die an Mitteln zur Querverbindung zwischen ihnen befestigt sind, und zwei Wirbelsäulenstäbe (1), die so angepasst sind, dass sie in Körpern (8, 31) der Haken aufgenommen werden können und um sich entlang eines Wirbelsäulensegments (S) zu erstrecken,
**dadurch gekennzeichnet, dass**
der Körper (8, 31) der Haken (2, 27, 54, 61) aus einem Schaft (11, 35) gebildet wird, der so angepasst ist, dass er auf querverlaufenden Knochenfortsätzen (5) des Wirbels (T) zur Auflage kommt, die vorher reseziert wurden, und
dadurch, dass die Verbindungsmittel für jedes Paar von Haken aus einem Paar von parallelen, fest miteinander verbundenen Stäben (6a, 6b) gebildet werden, die elastisch gewölbt sind, wobei sie einen querverlaufenden Bogen (6, 26, 51) bilden, dessen Enden (6c) in Bohrungen (13) eingreifen, die in den Haken (2) angeordnet sind, und
dadurch, dass die Mittel zum Befestigen der Haken an den Enden (6c) der Stäbe des querverlaufenden Bogens vorgesehen sind in Positionen, die elastische Rückführdrehmomente durch den querverlaufenden Bogen (6, 26, 51) der Haken erzeugen, die auf den querverlaufenden Knochenfortsätzen aufliegen.

2. Vorrichtung nach Anspruch 1,
**dadurch gekennzeichnet, dass**
die Bohrungen (13) einen länglichen Abschnitt aufweisen, der zu demjenigen der Stäbe (6a, 6b) des querverlaufenden Bogens (6) komplementär ist.

3. Vorrichtung nach Anspruch 2,
**dadurch gekennzeichnet, dass**
der Körper (8) jedes Hakens (2) ausgebildet ist, um den entsprechenden Wirbelsäulenstab (1) und einen seitlichen Schaft (11) aufzunehmen, der angepasst ist, um auf dem querverlaufenden Knochenfortsatz (5) zur Auflage zu kommen, dass die längliche Bohrung (13) an der Verbindungsstelle zwischen dem Körper und dem Schaft ausgespart ist, um von dem Ende (6c) des querverlaufenden Bogens (6) durchquert zu werden, und dass ein mit Gewinden versehenes Loch (14), das in dem Schaft (11) angeordnet ist, in ein längliches Loch (13) mündet, wobei das mit Gewinden versehene Loch angepasst ist, um mit einer Schraube (17) zum Sichern des Hakens auf dem querverlaufenden Bogen ausgestattet zu werden.

4. Vorrichtung nach einem der Ansprüche 1 bis 3,
**dadurch gekennzeichnet, dass**
die zwei Stäbe (6a) des querverlaufenden Bogens (6) durch Verschweißen (16) fest miteinander verbunden sind.

5. Vorrichtung nach Anspruch 3,
**dadurch gekennzeichnet, dass**
der Schaft (11) mit einem gekrümmten Ende (12) versehen ist, das einen Arm bildet, der angepasst ist, um sich entlang des querverlaufenden resezierten Knochenfortsatzes (5) zu erstrecken, während der Rest des Schafts angeordnet ist, um auf der stumpfen Fläche (4) des querverlaufenden Knochenfortsatzes (5) aufzuliegen.

6. Vorrichtung nach einem der Ansprüche 1 bis 5,
**dadurch gekennzeichnet, dass**
sie mit einem sekundären Bogen (22) ausgestattet ist, der aus einem im Wesentlichen U-förmigen Draht gebildet wird, der angepasst ist, um unter der Dornfortsatz-Knochenleiste (7) des Wirbels (T) hindurchzuführen, und dessen Schenkel (22a) unter den Körpern (8) der Haken (2) zur Auflage kommen, wobei auf den querverlaufenden Bogen Kräftepaare ausgeübt werden, die es ermöglichen, ihn auf der Dornfortsatz-Knochenleiste zu positionieren.

7. Vorrichtung nach Anspruch 6,
**dadurch gekennzeichnet, dass**
die Schenkel (22a) des sekundären Bogens (22) auf den seitlichen Auswüchsen (23) der Körper (8) der Haken (2) aufliegen und dort durch Sicherungsmittel gehalten werden, wie beispielsweise Schrauben (24), die in die Auswüchse eingesetzt werden.

8. Vorrichtung nach Anspruch 3,
**dadurch gekennzeichnet, dass**
jeder Haken (2) mit wenigstens einer Spitze (25) versehen ist, die aus seinem Schaft (11) hervorragt, um in den querverlaufenden Knochenfortsatz (5) einzudringen.

9. Vorrichtung nach einem der Ansprüche 1 bis 8,
**dadurch gekennzeichnet, dass**
der querverlaufende Bogen (26) eine dreifache Krümmung in einer horizontalen Ebene aufweist, um sich den anatomischen Formen des rückwärtigen Bogens des Wirbels anzupassen, und zwar einen mittleren konkaven Teil (26b) zwischen den geradlinigen Enden (26a), die in die jeweiligen Haken (27) eingeführt sind, und zwei seitliche konvexe Teile (26c) für die Verbindung mit den Enden.

10. Vorrichtung nach Anspruch 9,
**dadurch gekennzeichnet, dass**
jedes Ende (26a) des querverlaufenden Bogens (26) in einer Bohrung (28) gelagert ist, die in einem Schenkel (29) des Körpers (31) des Hakens (27) ausgespart ist, wobei dieser Körper seitlich durch einen Schaft (35) so verlängert ist, dass der Körper, wenn er einmal auf dem Wirbel (T) befestigt ist, auf der resezierten Fläche (4) des quer verlaufenden Knochenfortsatzes (5) aufliegt, und der Schaft sich an diesem entlang erstreckt.

11. Vorrichtung nach Anspruch 10,
**dadurch gekennzeichnet, dass**
in dem Schenkel (29) des Körpers (31) ein mit Gewinden versehenes Loch (44) angeordnet ist, das in die Bohrung (28) mündet und angepasst ist, um eine Schraube (45) zum Sichern des Hakens (27) auf dem querverlaufenden Bogen (26) aufzunehmen.

12. Vorrichtung nach Anspruch 10,
**dadurch gekennzeichnet, dass**
der querverlaufende Bogen (26) aus zwei Metalldrähten (38, 39) ausgebildet ist, von denen wenigstens einer (39) ein Gewindeende (41) umfasst, das sich über das Ende des unmittelbar angrenzenden Drahts (38) hinaus so erstreckt, dass es nach außen aus der Bohrung (28) des Körpers des Hakens hinausragt, und dieses Gewindeende ist mit einer Mutter (43) zum Sichern des Hakens auf dem querverlaufenden Bogen (26) ausgestattet.

13. Vorrichtung nach Anspruch 5,
**dadurch gekennzeichnet, dass**
der querverlaufende Bogen (51) aus drei Stäben gebildet wird, wobei zwei lange äußere Stäbe (52) vorhanden sind und ein mittlerer kurzer Stab (53), der mit den beiden anderen verschweißt ist, und
dadurch, dass der Haken (54) mit einer Schraube (55) ausgestattet ist, die positioniert ist, um eine seitliche Verstärkung (57) des Körpers des Hakens zu durchqueren, und
dass der entsprechende quer verlaufende Knochenfortsatz (5) mit seiner Spitze (55a) zum Anschlag in einer Vertiefung (59) des gekrümmten Endes (12) kommt, das den Arm des Hakens bildet.

14. Vorrichtung nach Anspruch 5,
**dadurch gekennzeichnet, dass**
wenigstens gewisse Haken (61) mit zwei Plättchen (62) ausgestattet sind, die in bezug auf eine Symmetrie-Ebene (P) des Hakens symmetrisch angeordnet sind, und dass sie sich ausgehend von seinem Schaft (11) zwischen seinem Körper (63) und dem zurückgebogenen Arm (12) erstrecken.

## Claims

1. Posterior spinal osteosynthesis device comprising at least two pairs of ratchets (2, 27, 54, 61) bearing laterally on vertebrae (T) fixed to means for transversely connecting them to each other, and two vertebral rods (1) adapted to be received in bodies (8, 31) of the hooks and to extend along a spinal segment (S), **characterized in that** the body (8, 31) of the hooks (2, 27, 54, 61) is designed as a stem (11, 35) adapted to bear on transverse processes (5) of the vertebra (T) which have been resected beforehand, and **in that** said connection means consist, for each pair of hooks, of a pair of parallel rods (6a, 6b) which are joined together and bent elastically to form a transverse arc (6, 26, 51) whose ends (6c) engage in bores (13) formed in the hooks (2), and **in that** means are provided for fixing the hooks to the ends (6c) of the rods of the transverse arc in positions generating elastic restoring couples via the transverse arc (6, 26, 51) of the hooks bearing on the transverse processes.

2. Device according to Claim 1, **characterized in that** the bores (13) have an oblong cross section complementing that of the rods (6a, 6b) of the transverse arc (6).

3. Device according to Claim 2, **characterized in that** the body (8) of each hook (2) is designed to receive the corresponding vertebral rod (1), and a lateral stem (11) adapted to be able to bear on the transverse process (5), the oblong bore (13) is formed at the junction between the body and the stem so as to be traversed by the end (6c) of the transverse arc (6), and a threaded hole (14) formed in the stem (11) opens into the oblong hole (13), this threaded hole being adapted to be equipped with a screw (17) for blocking the hook on the transverse arc.

4. Device according to one of Claims 1 to 3, **characterized in that** the two rods (6a) of the transverse arc (6) are joined together by welding (16).

5. Device according to Claim 3, **characterized in that** the stem (11) is provided with a curved end (12) forming a blade, adapted to extend along the resected transverse process (5), while the rest of the stem is designed to bear on the truncated face (4) of the transverse process (5).

6. Device according to one of Claims 1 to 5, **characterized in that** it is provided with a secondary arc (22) consisting of a substantially U-shaped wire adapted to pass under the spinous process (7) of the vertebra (T), and of which the branches (22a) bear under the bodies (8) of the hooks (2) and exert on the transverse arc couples which allow it to be pressed against the spinous process.

7. Device according to Claim 6, **characterized in that** the branches (22a) of the secondary arc (22) bear on lateral extensions (23) of the bodies (8) of the hooks (2) and are held there by blocking means such as screws (24) inserted into said extensions.

8. Device according to Claim 3, **characterized in that** each hook (2) is provided with at least one point (25) projecting from its stem (11) in order to be able to engage in the transverse process (5).

9. Device according to one of Claims 1 to 8, **characterized in that** the transverse arc (26) has a triple curvature in a horizontal plane in order to adapt to the anatomical shapes of the posterior arch of the vertebra, namely, between rectilinear ends (26a) inserted in the respective hooks (27), a concave central part (26b) and two convex lateral parts (26c) for connection at the ends.

10. Device according to Claim 9, **characterized in that** each end (26a) of the transverse arc (26) is lodged in a bore (28) formed in a branch (29) of the body (31) of the hook (27), this body being continued laterally by a stem (35) in such a way that once fitted on the vertebra (T) the body bears on the resected face (4) of the transverse process (5) and the stem extends along the latter.

11. Device according to Claim 10, **characterized in that** in said branch (29) of the body (31) there is a threaded hole (44) opening into the bore (28) and adapted to receive a screw (45) for blocking the hook (27) on the transverse arc (26).

12. Device according to Claim 10, **characterized in that** the transverse arc (26) is made up of two metal wires (38, 39), of which at least one (39) includes a threaded end (41) extending beyond the end of the contiguous wire (38) in such a way as to protrude outside the bore (28) of the body of the hook, and this threaded end is provided with a nut (43) for blocking the hook on the transverse arc (26).

13. Device according to Claim 5, **characterized in that** the transverse arc (51) consists of three rods, namely two long outer rods (52) and one short central rod (53) welded to the other two, and **in that** the hook (54) is equipped with a screw (55) positioned to traverse a lateral reinforcement (57) of the body of the hook and the corresponding transverse process (5), with its point (55a) coming into abutment in a recess (59) in the curved end (12) forming the blade of the hook.

14. Device according to Claim 5, **characterized in that** at least some hooks (61) are provided with two panels (62) arranged symmetrically with respect to a plane of symmetry (P) of the hook, and which extend from its stem (11) between its body (63) and the curved blade (12).
